(19) Europäisches Patentamt
European Patent Office
Office européen des brevets

(11) Publication number: 0 537 580 A2

# (12) EUROPEAN PATENT APPLICATION

(21) Application number: 92116943.9

(22) Date of filing: 05.10.92

(51) Int. Cl.5: C07D 401/04, C07D 401/14, A01N 43/56

(30) Priority: 18.10.91 JP 297772/91

(43) Date of publication of application: 21.04.93 Bulletin 93/16

(84) Designated Contracting States: BE CH DE ES FR GB IT LI NL

(71) Applicant: NIHON BAYER AGROCHEM K.K.
7-1, Nihonbashi Honcho 2-chome Chuo-ku
Tokyo 103(JP)

(72) Inventor: Tsuboi, Shin-ichi
1-2-3, Cho-cho
Oyama-shi, Tochigi(JP)
Inventor: Wada, Katsuaki
7-11-20, Jyoto
Oyama-shi, Tochigi(JP)
Inventor: Maurer, Fritz
Roeberstrasse 8
W-5600 Wuppertal 1(DE)
Inventor: Hattori, Yumi
12397-4, Yuki
Oyama-shi, Tochigi(JP)
Inventor: Sone, Shinzaburo
11758-32, Yuki
Oyama-shi, Tochigi(JP)

(74) Representative: Schumacher, Günter, Dr. et al
c/o Bayer AG Konzernverwaltung RP
Patentabteilung
W-5090 Leverkusen 1 Bayerwerk (DE)

## (54) Pyrazolines as insecticides.

(57) Novel pyrazolines of the formula (I)

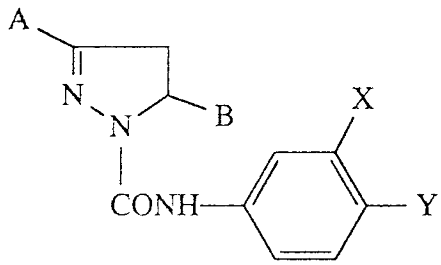

wherein A represents a phenyl group which may be substituted by at least one substituent selected from the class consisting of halogen, $C_{1-4}$alkyl, $C_{1-4}$alkoxy and halogeno-$C_{1-4}$alkyl, or A represents halogen-substituted 3-pyridyl,
B represents phenyl which may be substituted by halogen, or B represents halogen substituted 3-pyridyl,
X represents hydrogen, halogen or halogeno $C_{1-4}$alkyl, and
Y represents hydrogen, halogen, $C_{1-4}$alkyl or halogeno-$C_{1-4}$alkyl, with the proviso that X and Y do not simultaneously represent hydrogen atom, and that at least one of A and B represents halogen-substituted 3-pyridyl.

The new compounds exhibit powerful insecticidal properties.

EP 0 537 580 A2

The present invention relates to novel pyrazolines, to a process for the preparation, to their use as insecticides, as well as novel intermediates for their preparation and to a process for the preparation of those intermediates.

It has already been disclosed that a certain group of pyrazolines is useful as insecticides (see, Japanese Laid-Open patent application No. 87028/1973, which is an equivalent of DE-A 2,304,586 and of US Patent Nos. 3,991,073; 4,010,271; 4,095,026).

There have been found novel pyrazolines of the formula (I)

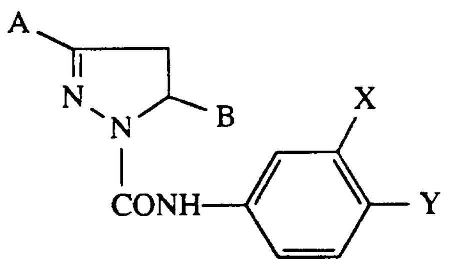

(I),

wherein A represents a phenyl which may be substituted by at least one substituent selected from the class consisting of halogen, $C_{1-4}$-alkyl, $C_{1-4}$-alkoxy and halogeno-$C_{1-4}$-alkyl, or A represents halogen-substituted 3-pyridyl,

B represents phenyl which may be substituted by halogen, or B represents halogen-substituted 3-pyridyl,

X represents hydrogen, halogen or halogeno-$C_{1-4}$-alkyl, and Y represents hydrogen, halogen, $C_{1-4}$-alkyl or halogeno-$C_{1-4}$-alkyl, with the proviso that X and Y do not simultaneously represent hydrogen and that at least one of A and B represents halogen-substituted 3-pyridyl.

The pyrazolines of the formula (I) are obtained when

a) pyrazolines of the formula (II)

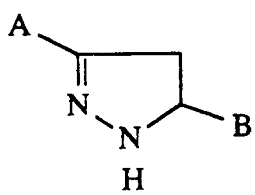

(II),

wherein A and B have the same meanings as mentioned above,

are reacted with phenyl isocyanates of the formula (III)

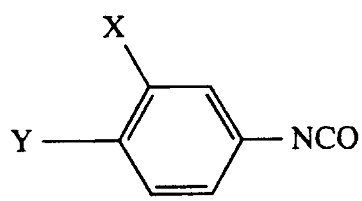

(III),

wherein X and Y have the same meanings as mentioned above,

in the presence of inert solvents.

The novel pyrazolines of the formula (I) exhibit powerful insecticidal properties.

Surprisingly, the pyrazolines according to the invention exhibit a substantially greater insecticidal action than those known from the prior art of the above-mentioned Japanese Laid-Open patent application. Particularly, the compounds according to the invention exhibit excellent insecticidal effects against Lepidoptera.

Referring to the formula (I) according to the invention, as well as the formula (II) and (III) of the intermediates used of the production thereof, halogen includes fluorine, chlorine, bromine and iodine, preferably fluorine, chlorine and bromine.

$C_{1-4}$-alkyl and $C_{1-4}$-alkyl moieties contained in $C_{1-4}$alkoxy and in halogeno-$C_{1-4}$-alkyl include methyl, ethyl, n-propyl, isopropyl, and n-(or iso-, sec- or tert-)butyl, preferably methyl, ethyl, n-propyl and isopropyl.

Among the pyrazolines according to the invention, of the formula (I), preferred compounds are those in which
A represents phenyl which may be substituted by at least one substituent selected from the class consisting of fluorine, chlorine, bromine, $C_{1-4}$-alkyl, $C_{1-4}$-alkoxy, difluoromethyl and trifluoromethyl, or A represents fluorine-, chlorine- or bromine-substituted 3-pyridyl,
B represents phenyl which may be substituted by fluorine, chlorine, bromine, or B represents fluorine-, chlorine- or bromine-substituted 3-pyridyl,
X represents hydrogen, chlorine or trifluoromethyl, and Y represents hydrogen, halogen, $C_{1-4}$-alkyl or fluoro-substituted methyl and with the proviso that X and Y do not simultaneously represent hydrogen atom, and that at least one of A and B represents halogen-substituted 3-pyridyl.

Very particularly preferred pyrazolines of the formula (I) are those in which
A represents phenyl which may be substituted by at least one substituent selected from the class consisting of fluorine, chlorine, methyl, isopropyl, tert.-butyl, methoxy, trifluoromethyl or difluoromethyl, or
A represents chlorine- or bromine-substituted 3-pyridyl,
B represents chlorine- or bromine-substituted 3-pyridyl,
X represents hydrogen, chlorine or trifluoromethyl, and
Y represents fluorine, chlorine, iodine,methyl, ethyl, isopropyl, tert.-butyl difluoromethyl or trifluoromethyl.

In addition to the compounds disclosed in the Preparation Examples the following compounds of general formula (I) are mentioned:

Table 1

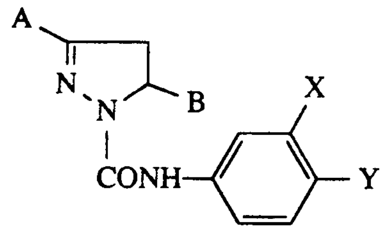

| A | B | X | Y |
|---|---|---|---|
| phenyl | 6-Cl-3-pyrdiyl | H | Cl |
| " | " | H | $CF_3$ |
| " | " | Cl | $CF_3$ |
| 4-F-phenyl | " | H | F |
| " | " | H | Cl |
| " | " | H | I |
| " | " | H | $CH_3$ |
| " | " | H | $C_2H_5$ |
| " | " | H | $C_3H_7$-iso |
| " | " | H | $C_4H_9$-tert |
| " | " | H | $CF_3$ |
| " | " | H | $CHF_2$ |
| " | " | Cl | $CF_3$ |
| " | " | $CF_3$ | H |
| " | " | $CF_3$ | $CF_3$ |
| 4-Cl-phenyl | " | H | F |
| " | " | H | Cl |
| " | " | H | I |
| " | " | H | $CH_3$ |

Table 1 (continued)

| A | B | X | Y |
|---|---|---|---|
| 4-Cl-phenyl | 6-Cl-3-pyridyl | H | $C_2H_5$ |
| " | " | H | $C_3H_7$-iso |
| " | " | H | $C_4H_9$-tert |
| " | " | H | $CF_3$ |
| " | " | H | $CHF_2$ |
| " | " | Cl | $CF_3$ |
| " | " | $CF_3$ | H |
| " | " | $CF_3$ | $CF_3$ |
| 3,4-$Cl_2$-phenyl | " | H | Cl |
| " | " | H | $CF_3$ |
| " | " | Cl | $CF_3$ |
| 4-$CH_3$-phenyl | " | H | $CF_3$ |
| 4-iso-$C_3H_7$-phenyl | " | H | $CF_3$ |
| 4-tert-$C_4H_9$-phenyl | " | H | $CF_3$ |
| 4-$CH_3O$-phenyl | " | H | $CF_3$ |
| 6-Cl-3-pyridyl | " | H | Cl |
| " | " | H | $CF_3$ |
| " | " | Cl | $CF_3$ |
| 4-F-phenyl | 5,6-$Cl_2$-pyridyl | H | Cl |
| " | " | H | $CF_3$ |
| 4-Cl-phenyl | " | H | Cl |

Table 1 (continued)

| A | B | X | Y |
|---|---|---|---|
| 4-F-phenyl | 5-Br-3-pyridyl | H | Cl |
| " | " | H | $CF_3$ |
| 4-Cl-phenyl | 2-Cl-3-pyridyl | H | Cl |
| " | " | H | $CF_3$ |
| 6-Cl-3-pyridyl | phenyl | H | Cl |
| " | " | H | $CF_3$ |
| " | " | H | $CHF_2$ |
| " | " | Cl | $CF_3$ |
| " | 4-F-phenyl | H | Cl |
| " | " | H | $CF_3$ |
| " | " | H | $CHF_2$ |
| " | " | Cl | $CF_3$ |
| " | 4-Cl-phenyl | H | Cl |
| " | " | H | $CF_3$ |
| " | " | H | $CHF_2$ |
| " | " | Cl | $CF_3$ |
| 4-$CF_3$-phenyl | 6-Cl-3-pyridyl | H | $CF_3$ |
| " | " | Cl | $CF_3$ |
| 4-$CHF_2$-phenyl | " | H | $CF_3$ |
| " | " | Cl | $CF_3$ |

If, for example, 3-phenyl-5-(6-chloro-3-pyridyl)-2-pyrazoline and 4-chlorophenyl isocyanate are used as starting materials, the course of the reaction can be represented by the following equation:

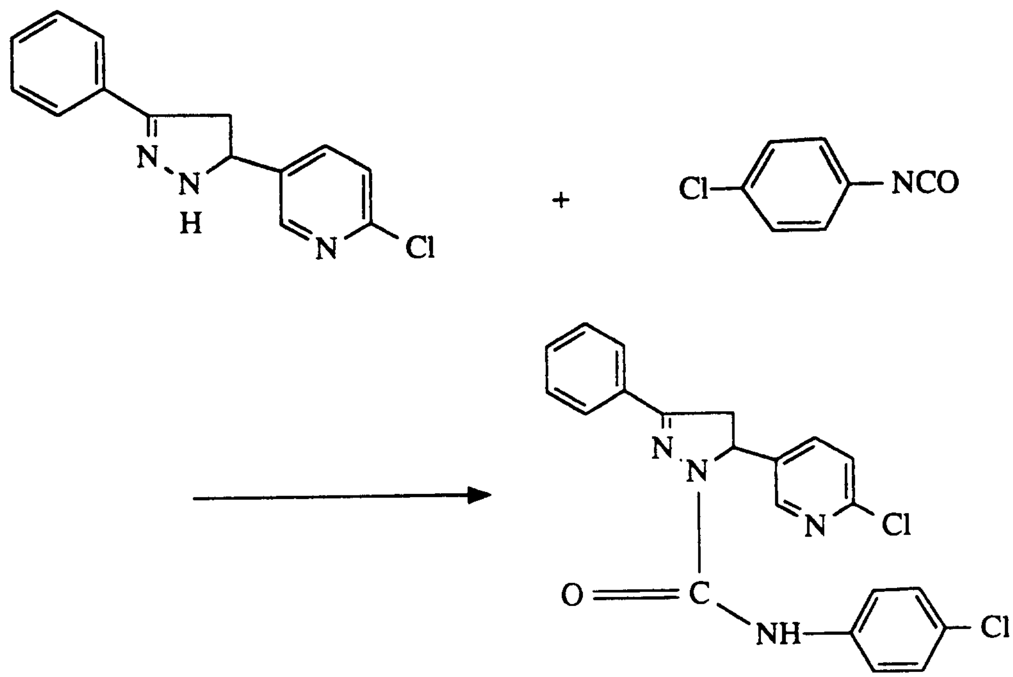

In process a), the starting material of the formula (II) means compounds based on the above definitions of A and B, preferably compounds based on the above preferred definitions.

The starting materials of the formula (II) are novel compounds, and can be obtained in analogy to the process described in Japanese Laid-Open patent application No. 87028/1973, as illustrated in detail in the example below.

The starting materials of the formula (II) are obtained when the compounds of the formula (IV)

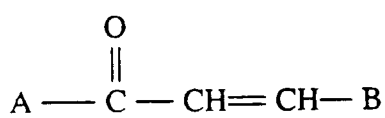

(IV),

wherein A and B have the meanings stated above,
are reacted with hydrazine hydrate,
in the presence of inert solvents.

The compounds represented by the formula (IV) are novel compounds too, and can be obtained, for instance, when
the compounds of the following formula (V)

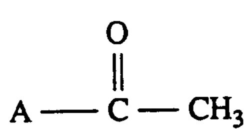

(V),

wherein A has meaning stated above,
are reacted with the compound of the following formula (VI)

$$B - CHO \qquad (VI),$$

wherein B has the meaning stated above,
if appropriate in the presence of acid binding substances and in the presence of inert solvents.

The compounds represented by the formula (V) and (VI) are compounds known in the field of organic chemistry.

As representative examples of starting compounds of the formula (II) may be mentioned:

3-phenyl-5-(6-chloro-3-pyridyl)-2-pyrazoline,
3-(4-fluorophenyl)-5-(6-chloro-3-pyridyl)-2-pyrazoline
3-(4-chlorophenyl)-5-(6-chloro-3-pyridyl)-2-pyrazoline,
3-(6-chloro-3-pyridyl)-5-phenyl-2-pyrazoline,
3-(6-chloro-3-pyridyl)-5-(4-fluorophenyl)-2-pyrazoline, and
3-(6-chloro-3-pyridyl)-5-(4-chlorophenyl)-2-pyrazoline.

In process a), the starting compounds of the formula (III) mean compounds based on the above definitions of X and Y, preferably compounds based on the above preferred definitions.

The starting compounds are well known phenyl isocyanates. Representative examples of the formula (II) include:

4-chlorophenyl isocyanate,
4-trifluoromethylphenyl isocyanate,
4-difluoromethylphenyl isocyanate,
3-chlorophenyl isocyanate, and
3-chloro-4-trifluoromethylphenyl isocyanate.

In carrying out the process a) mentioned above, any inert solvent can be used as suitable diluent.

Examples of such diluents are water; aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, ethylene chloride, chlorobenzene, dichlorobenzene, and the like; ethers such as diethyl ether, methylethyl ether, di-isopropyl ether, di-butyl ether, propylene oxide, dimethoxyethane (DME), dioxane, tetrahydrofurane (THF) and the like; ketones such as acetone methylethyl ketone (MEK), methyl-iso-propyl ketone, methyl-iso-butyl ketone (MIBK) and the like; nitriles such as acetonitrile, propionitrile, acrylonitrile, and the like; alcohols such as methanol, ethanol, iso-propanol, butanol, ethylene glycol and the like; esters such as ethyl acetate, amyl acetate, and the like; acid amides such as dimethyl formamide, diethyl acetamide, and the like; sulfones and sulfoxides such as dimethyl sulfoxide, sulfolane and the like; and, bases, for example, such as pyridine.

In the above mentioned process a), the reaction temperature can be varied within a substantially wide range. In general, the reaction is carried out at a temperature of from about 0 °C to about 120 °C, preferably from 10 °C to about 40 °C.

Further, the reaction is preferably carried out under normal pressure, although it is also possible to employ a higher or reduced pressure.

When the above mentioned process a) according to the present invention is carried out, use is made, for example, of the above mentioned compound (III) in the amount from 1.0 to 1.5 mols, preferably 1 to 1.2 mols, per one mol of the above mentioned compound (II), optionally in the presence of inert solvents such as acetonitrile, for example, to obtain the desired compounds of the formula (I).

The active compounds of the formula (I) are well tolerated by plants, have a favorable level of toxicity to warm-blooded animals, and can be used for combating arthropod pests, especially insects which are encountered in agriculture, in forestry, in the protection of stored products and of materials, and in the hygiene field. They are active against normally sensitive and resistant species and against all or some stages of development. The above-mentioned pests include:

from the class of the Isopoda, for example, Oniscus Asellus, Armadillidium vulgare and Porcellio scaber;
from the class of the Diplopoda, for example, Blaniulus guttulatus;
from the class of the Chilopoda, for example, Geophilus carpophagus and Scutigera spec.;
from the class of the Symphyla, for example, Scuti gerella immaculata;
from the order of the Thysanura, for example, Lepisma saccharina;
from the order of the Collembola, for example, Onychiurus armatus;
from the order of the Orthoptera; for example, Blatta orientalis, Periplaneta americana. Leucophaea maderae, Blattella germanica, Acheta domesticus, Gryllotalpa spp., Locusta migrato ria migratorioides, Melanoplus differentialis and Schistocerca gregaria;
from the order of the Dermaptera, for example, Forficula auricularia;
from the order of the Isoptera, for example, Reticulitermes spp.;
from the order of the Anoplura, for example, Phylloxera vastatrix, Pemphigus spp., Pediculus humanus corporis, Haematopinus spp. and Linognathus spp.;
from the order of the Mallophaga, for example, Trichodectes spp. and Damalinea spp.;
from the order of the Thysanoptera, for example, Hercinothrips femoralis and Thrips tabaci;

from the order of the Heteroptera, for example, Eurygaster spp., Dysdercus intermedius, Piesma guadrata, Cimex lectularius, Rhodnius prolixus and Triatoma spp.;
from the order of the Homoptera, for example, Aleurodes brassicae, Bemisia tabaci, Trialeurodes vaporariotum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. and Psylla spp.;
from the order of the Lepidoptera, for example, Pectinophora gossypiella, Bupalus piniarius, Cheimatobia brumata, Lithocolletis blancardella, Hyponomeuta padella, Plutella maculipennis Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella Homona magnanima and Tortrix viridana;
from the order of the Coleoptera, for example, Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorr hynchus sulcatus, Cosmopolites sordidus, Ceuthorthynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis and Costelytra zealandica;
from the order of the Hymenoptera, for example, Diprion spp., Hoplocampa spp., Lasius spp., Monomorium pharaonis and Vespa spp.;
from the order of the Diptera, for example, Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae and Tipula paludosa.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with bunning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents, diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may he used ground natural minerals, such as kaolins, clays talc, chalk, quartz, attapulgite, montmorillonite or diatomaceous earth, and ground synthetic minerals, such as highly-dispersed silicic acid, alumina and silicates. As solid carriers for granules there may he used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese, boron, copper, cobalt, molybdenum and zinc.

The formulations in general contain from 0.1 to 95 percent by weight of active compound, preferably from 0.5 to 90 percent by weight.

The active compounds according to the invention can be present in their commercially available formulations and in the use forms, prepared from these formulations, as a mixture with other active compounds, such as insecticides, baits, sterilising agents, acaricides, nematicides, fungicides, growth-regulating substances or herbicides. The insecticides include, for example, phosphates, carbamates, carboxylates, chlorinated hydrocarbons, phenylureas, substances produced by microorganisms.

The active compounds according to the invention can furthermore be present in their commerically available formulations and in the use forms, prepared from these formulations, as a mixture with synergistic agents. Synergistic agent are compounds which increase the action of the active compounds, without it being necessary for the synergistic agent added to be active itself.

The active compound content of the use forms prepared from the commercially available formulations can vary within wide limits. The active compound concentration of the use forms can be from 0.0000001 to 100% by weight of active compound, preferably between 0.0001 and 1% by weight.

The compounds are employed in a customary manner appropriate for the use forms.

When used against hygiene pests and pests of stored products, the active compounds are distinguished by an excellent residual action on wood and clay as well as a good stability to alkali on limed substrates.

The preparation and use of the active compounds according to the invention can be seen from the following examples.

Example 1

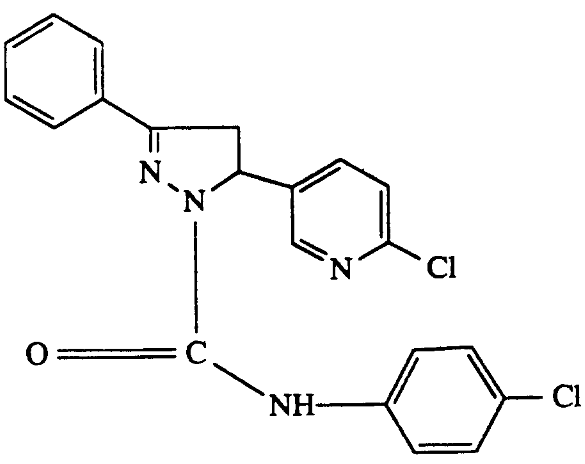

(Compound No I.1)

3-Phenyl-5-(6-chloro-3-pyridyl)-2-pyrazoline (2.58 g) was dissolved in 10 ml of acetonitrile. The resulting solution was admixed with 4-chlorophenyl isocyanate (1.53 g) at room temperature, and stirred at room temperature for 10 hours, and then the solvent was distilled off under reduced pressure. The crude product thus formed was recrystallized from ethanol to give the 1-(4-chlorophenyl) carbamoyl-3-phenyl-5-(6-chloro-3-pyridyl)-2-pyrazoline (2.79 g). mp. 168-175 ° C.

Example 2

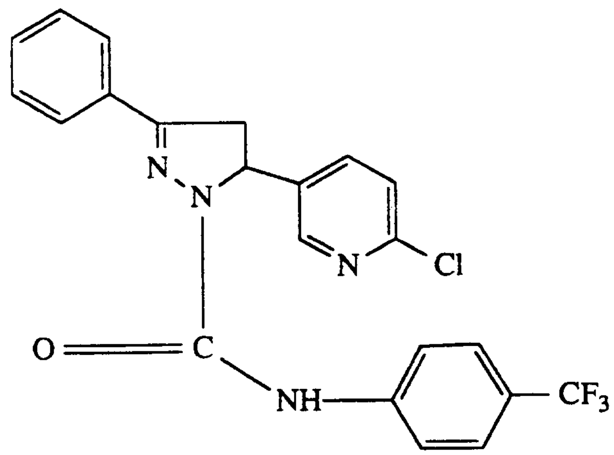

(Compound No I.2)

According to a procedure in analogy to that employed in Example 1, a reaction operation was carried out by using 4-trifluoromethylphenyl isocyanate, instead of 4-chlorophenyl isocyanate employed in Example 1, so that the 1-(4-trifluoromethylphenyl) carbamoyl-3-phenyl-5-(6-chloro-3-pyridyl)-2-pyrazoline (2.89 g) was produced.
mp. 159-162 ° C.

According to the same way as those employed in the above-mentioned Examples, a number of compounds can be obtained as specifically shown in Table 2, wherein the compounds prepared in the aforementioned Examples are also shown.

Table 2

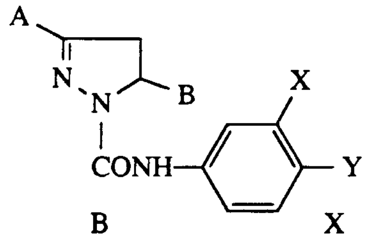

(I)

| Compound No. | A | B | X | Y | mp.°C |
|---|---|---|---|---|---|
| I.1 | phenyl | 6-Cl-3-pyridyl | H | Cl | 168-175 |
| I.2 | " | " | H | $CF_3$ | 159-162 |
| I.3 | 4-F-phenyl | " | H | $CF_3$ | 159-161 |
| I.4 | " | " | H | $CHF_2$ | 181-184 |
| I.5 | " | " | Cl | $CF_3$ | 178-181 |
| I.6 | 4-Cl-phenyl | " | H | Cl | 239-241 |
| I.7 | " | " | H | I | 233-236 |
| I.8 | " | " | H | $C_3H_7$-iso | 168-172 |
| I.9 | " | " | H | $CF_3$ | 214-216 |
| I.10 | " | " | H | $CHF_2$ | 227-230 |
| I.11 | " | " | Cl | $CF_3$ | 172-177 |
| I.12 | " | " | $CF_3$ | H | 226-227 |
| I.13 | 3,4-$Cl_2$-phenyl | " | H | Cl | 164-177 |
| I.14 | " | " | H | $CF_3$ | 142-162 |
| I.15 | 4-$CH_3$-phenyl | " | H | $CF_3$ | 248-250 |
| I.16 | 4-$CH_3O$-phenyl | " | H | $CF_3$ | 232-233 |
| I.17 | 4-Cl-phenyl | " | H | F | 204-206 |
| I.18 | " | " | H | $CH_3$ | 225-229 |
| I.19 | 6-Cl-3-pyridyl | 6-Cl-3-pyridyl | H | $CF_3$ | 187-188 |

Example 3 (synthesis of intermediate compound)

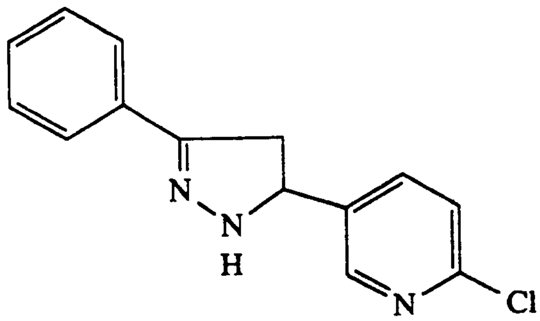

(Compound No II-1)

1-Phenyl-3-(6-chloro-3-pyridyl)-propenone (2.44 g) was dissolved in 20 ml of ethanol. The resultant solution was admixed with 0.5 ml of hydrazine hydrate, and heated under reflux for 3 hours. After the completion of the reaction, the solvent was distilled off under reduced pressure to give 3-phenyl-5-(6-chloro-3-pyridyl)-2-pyrazoline (2.58 g).

| | |
|---|---|
| $^1$H-NMR (90 MHz, δppm, $CDCl_3$) | 2.98 (1H, dd) |
| | 3.53 (1H, dd) |
| | 4.93 (1H, dd) |
| | 6.00 (1H, m) |
| | 7.24-7.72 (7H, m) |
| | 8.33 (1H, d) |

Example 4 (synthesis of starting compound)

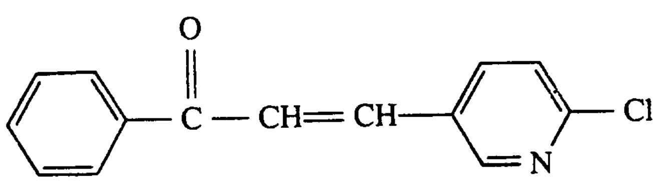

(Compound No. IV-1)

Acetophenone (12.0 g) and 6-chloro-nicotinic aldehyde (14.2 g) were dissolved in 100 ml of ethanol. To the resulting solution were dropwise added 20 ml of a 20% aqueous solution of sodium hydroxide at room temperature. The crystals thus formed were separated by filtration, washed with cold ethanol and dried in air to give 1-phenyl-3-(6-chloro-3-pyridyl)-propenone (19.9 g).
mp. 186-189 °C.

According to the same way as those employed in the above-mentioned Examples 3 and 4, a number of compounds can be obtained as specifically shown in Tables 3 and 4 respectively. These tables also show the compounds prepared in Examples 3 and 4.

Table 3

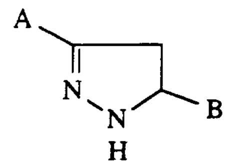

(II)

| Compound No. | A | B | $^1$H-NMR (90 MHz, $CDCl_3$, ppm) |
|---|---|---|---|
| II-1 | phenyl | 6-Cl-3-pyridyl | 2.98 (1H, dd)<br>3.53 (1H, dd)<br>4.93 (1H, dd)<br>6.00 (1H, m)<br>7.24-7.72 (7H, m)<br>8.33 (1H, d) |
| II-2 | 4-Cl-phenyl | 6-Cl-3-pyridyl | 2.93 (1H, dd)<br>3.50 (1H, dd)<br>4.96 (1H, dd)<br>5.20 (1H, m)<br>7.20-7.80 (6H, m)<br>8.33 (1H, d) |
| II-3 | 4-F-phenyl | 6-Cl-3-pyridyl | 2.90 (1H, dd)<br>3.50 (1H, dd)<br>4.95 (1H, dd)<br>6.00 (1H, m)<br>6.80-7.83 (6H, m)<br>8.33 (1H, d) |
| II-4 | 4-$CH_3$-phenyl | 6-Cl-3-pyridyl | 2.33 (3H, s)<br>2.90 (1H, dd)<br>3.50 (1H, dd)<br>4.88 (1H, dd)<br>5.87 (1H, m)<br>7.07-7.60 (5H, m)<br>7.70 (1H, dd)<br>8.33 (1H, d) |

Table 3 (continued)

| Compound No. | A | B<br>H | $^1$H-NMR (90 MHz, $CDCl_3$, ppm) |
|---|---|---|---|
| II-5 | 4-tert-$C_4H_9$phenyl | 6-Cl-3-pyridyl | 1.33 (9H, s)<br>2.98 (1H, dd)<br>3.53 (1H, dd)<br>4.93 (1H, dd)<br>6.00 (1H, m)<br>7.20-7.75 (6H, m)<br>8.33 (1H, d) |
| II-6 | 6-Cl-3-pyridyl | 6-Cl-3-pyridyl | 2.95 (1H, dd)<br>3.57 (1H, dd)<br>5.03 (1H, dd)<br>6.47 (1H, m)<br>7.30 (1H, d)<br>7.33 (1H, d)<br>7.77 (1H, dd)<br>7.97 (1H, dd)<br>8.35 (1H, dd)<br>8.48 (1H, d) |
| II-7 | 6-Cl-3-pyridyl | 4-Cl-phenyl | 2.93 (1H, dd)<br>3.45 (1H, dd)<br>4.90 (1H, dd)<br>6.22 (1H, m)<br>7.20-7.35 (4H, m)<br>7.93 (1H, dd)<br>8.45 (1H, d) |
| II-8 | 3,4-$Cl_2$-phenyl | 6-Cl-3-pyridyl | oil |

Table 4

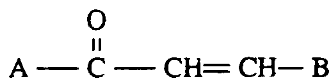 (IV)

| Compound No. | A | B | mp °C |
|---|---|---|---|
| IV-1 | phenyl | 6-Cl-3-pyridyl | 186-189 |
| IV-2 | 4-F-phenyl | 6-Cl-3-pyridyl | 152-154 |
| IV-3 | 4-Cl-phenyl | 6-Cl-3-pyridyl | 173-175 |
| IV-4 | 4-$CH_3$-phenyl | 6-Cl-3-pyridyl | 180-182 |
| IV-5 | 4-tert-$C_4H_9$-phenyl | 6-Cl-3-pyridyl | 165-166 |
| IV-6 | 3,4-$Cl_2$-phenyl | 6-Cl-3-pyridyl | 122-123 |
| IV-7 | 4-Cl-3-pyridyl | 6-Cl-3-pyridyl | 227-228 |
| IV-8 | 4-Cl-3-pyridyl | 4-Cl-phenyl | 168-169 |

Biotest Example:

Comparative compounds

C-1:

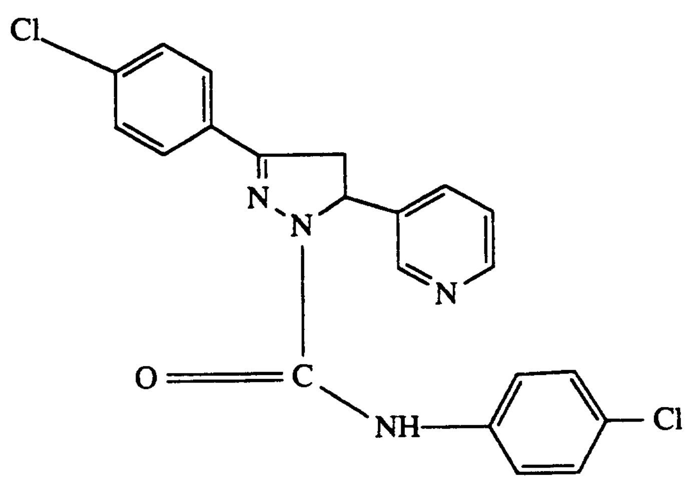

(disclosed in Japanese Laid-open Patent Application No. 87028/1973)

C-2:

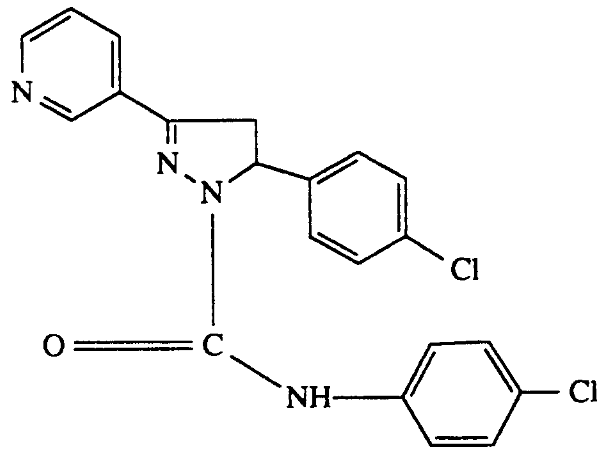

(also disclosed in Japanese Laid-open Patent Application No. 87028/1973)

Test Example 1

Biotest carried out against larvae of Spodoptera litura

Preparation of test formulation:

solvent: 3 parts by weight of xylene
Emuslifier: 1 part by weight of polyoxyethylene-alkylphenyl-ether

To prepare suitable formulations of active compounds, 1 part by weight of each of the active compounds was mixed with the above-mentioned amount of the emulsifier, and the mixture was diluted with water to the predetermined concentration.

Test Method:

Leaves of sweet potato plants (Ipomoea batatas Lam.) were soaked in a diluted aqueous solution of an active compound having a predetermined concentration, dried in air and placed on a dish with a diameter of 9 cm. 10 pieces of 3-instar larvae of Spodoptera litura were released into the dish, which was then placed in a green house having a constant temperature of 28° C. After 7 days, the number of the killed insects was determined to obtain the mortality of the insects.

The results are shown in Table 5.

Table 5

| Compound No. | Concentration of active compound (ppm) | Insect mortality after 7 days (%) |
|---|---|---|
| I.3 | 40 | 100 |
| I.4 | 40 | 100 |
| I.5 | 40 | 100 |
| I.9 | 40 | 100 |
| I.12 | 40 | 100 |
| control | | |
| C-1 | 1000 | 0 |
| C-2 | 1000 | 0 |

## Claims

1. Pyrazolines of the formula (I)

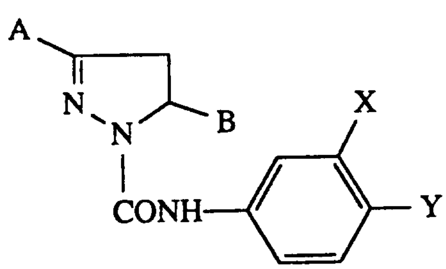

(I),

wherein A represents phenyl which may be substituted by at least one substituent selected from the class consisting of halogen, $C_{1-4}$-alkyl, $C_{1-4}$-alkoxy and halogeno-$C_{1-4}$-alkyl, or A represents halogen-substituted 3-pyridyl.
B represents phenyl which may be substituted by halogen, or B represents halogen-substituted 3-pyridyl,
X represents hydrogen, halogen or halogeno-$C_{1-4}$-alkyl, and Y represents hydrogen, halogen, $C_{1-4}$-alkyl or halogeno-$C_{1-4}$-alkyl, with the proviso that X and Y do not simultaneously represent hydrogen and that at least one of A and B represents halogen-substituted 3-pyridyl.

2. The compounds of the claim 1 wherein A represents phenyl which may be substituted by at least one substituent selected from the class consisting of fluorine, chlorine, bromine, $C_{1-4}$-alkyl, $C_{1-4}$-alkoxy, difluoromethyl and trifluoromethyl, or A represents fluorine-, chlorine- or bromine-substituted 3-pyridyl,
B represents phenyl which may be substituted by fluorine, chlorine, bromine, or B represents fluorine-,

chlorine- or bromine-substituted 3-pyridyl,
X represents hydrogen, chlorine or trifluoromethyl, and Y represents hydrogen, halogen, $C_{1-4}$-alkyl or fluoro-substituted methyl and with the proviso that X and Y do not simultaneously represent hydrogen, and that at least one of A and B represents halogen-substituted 3-pyridyl.

**3.** The compounds of the claim 1) or 2) wherein A represents phenyl which may be substituted by at least one substituent selected from the class consisting of fluorine, chlorine, methyl, isopropyl, tert.-butyl, methoxy, trifluoromethyl or difluoromethyl or A represents chlorine- or bromine-substituted 3-pyridyl,
B represents chlorine- or bromine-substituted 3-pyridyl,
X represents hydrogen, chlorine or trifluoromethyl, and
Y represents fluorine, chlorine, iodine, methyl, ethyl, isopropyl, tert.-butyl, difluoromethyl or trifluoromethyl.

**4.** Process for the preparation of pyrazolines of the formula (I)

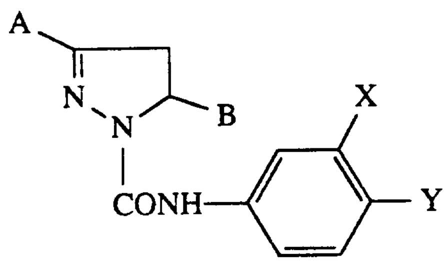

(I),

wherein A represents phenyl which may be substituted by at least one substituent selected from the class consisting of halogen, $C_{1-4}$-alkyl, $C_{1-4}$-alkoxy and halogeno-$C_{1-4}$-alkyl, or A represents halogen-substituted 3-pyridyl.
B represents phenyl which may be substituted by halogen, or B represents halogen-substituted 3-pyridyl,
X represents hydrogen, halogen or halogeno-$C_{1-4}$-alkyl, and Y represents hydrogen, halogen, $C_{1-4}$-alkyl or halogeno-$C_{1-4}$-alkyl, with the proviso that X and Y do not simultaneously represent hydrogen and that at least one of A and B represents halogen-substituted 3-pyridyl,
characterized in that
pyrazolines of the formula (II)

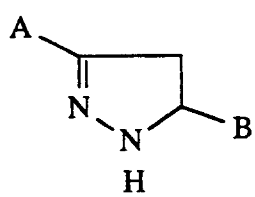

(II),

wherein A and B have the same meanings as mentioned above,
are reacted with phenyl isocyanates of the formula (III)

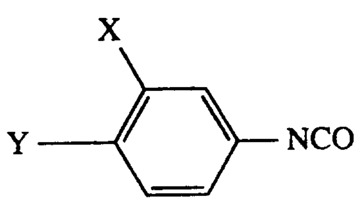

(III),

wherein X and Y have the same meanings as mentioned above,
in the presence of inert solvents.

5. Insecticidal compositions, characterized in that they contain at least one pyrazoline of the formula (I) according to claim 1.

6. A method of combating insects, characterized in that pyrazolines of the formula (I) according to claim 1 are allowed to act on insects and/or their habitat.

7. Use of pyrazolines of the formula (I) according to claim 1 for combating insects.

8. Process for the preparation of insecticidal compositions, characterized in that pyrazolines of the formula (I) according to claim 1 are mixed with extenders and/or surface active agents.

9. Pyrazolines of the formula (II)

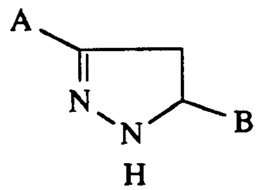

(II),

wherein A represents a phenyl group which may be substituted by at least one group selected from the class consisting of halogen, $C_{1-4}$-alkyl, $C_{1-4}$-alkoxy and halogeno-$C_{1-4}$-alkyl, or A represents halogen-substituted 3-pyridyl.
B represents phenyl which may be substituted by halogen, or B represents halogen-substituted 3-pyridyl, with the proviso that at least one of A and B represents halogen-substituted 3-pyridyl.

10. Process for the preparation of pyrazolines of the formula (II)

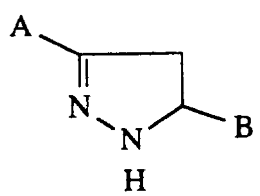

(II),

wherein A represents a phenyl group which may be substituted by at least one group selected from the class consisting of halogen, $C_{1-4}$-alkyl,$C_{1-4}$-alkoxy and halogeno-$C_{1-4}$-alkyl, or A represents halogen-substituted 3-pyridyl,
B represents phenyl which may be substituted by halogen, or B represents halogen-substituted 3-pyridyl, with the proviso that at least one of A and B represents halogen-substituted 3-pyridyl,
characterized in that
the compounds of the formula (IV)

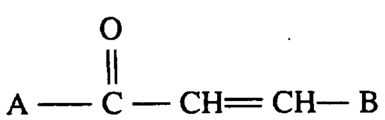

(IV),

wherein A represents phenyl which may be substituted by at least one substituent selected from the class consisting of halogen, $C_{1-4}$-alkyl, $C_{1-4}$-alkoxy and halogen-$C_{1-4}$-alkyl, or A represents halogen-substituted 3-pyridyl,
B represents phenyl which may be substituted by halogen, or B represents halogen-substituted 3-pyridyl, with the proviso that at least one of A and B represents halogen-substituted 3-pyridyl,
are reacted with hydrazine hydrate,

in the presence of inert solvents.

**11.** The compounds of the formula (IV)

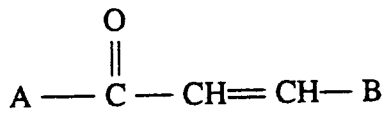

(IV),

wherein A represents phenyl which may be substituted by at least one substituent selected from the class consisting of halogen, $C_{1-4}$-alkyl, $C_{1-4}$-alkoxy and halogeno-$C_{1-4}$-alkyl, or A represents halogen-substituted 3-pyridyl,

B represents phenyl which may be substituted by halogen, or B represents halogen-substituted 3-pyridyl, with the proviso that at least one of A and B represents halogen-substituted 3-pyridyl,